# EUROPEAN PATENT SPECIFICATION

(11) **EP 1 225 876 B1**
(45) Date of publication and mention of the grant of the patent: **11.02.2004**
(21) Application number: 00974794.0
(22) Date of filing: 20.10.2000
(51) Int. Cl.: A61K 9/20

(54) **GASTRORESISTANT TABLETS FOR ALIMENTARY, DIETETIC AND THERAPEUTIC USE**
MAGENSAFTRESISTENTE TABLETTE FÜR NAHRUNG, DIÄT UND THERAPIE
COMPRIMES GASTRORESISTANTS A USAGE ALIMENTAIRE, DIETETIQUE ET THERAPEUTIQUE

(30) Priority: 21.10.1999 IT MI992206
(43) Date of publication of application: 31.07.2002
(73) Proprietor: Truffini & Regge' Farmaceutici Srl, 20134 Milano (IT)
(72) Inventor: Seneci, Alessandro, I-20090 Segrate (IT); Alberico, Pia, I-22100 Como (IT)
(74) Representative: Pistolesi, Roberto
(86) International application number: PCT/IT2000/000424
(87) International publication number: WO 2001/028526

(56) References cited:
- WO-A-95/05808
- WO-A-98/35656
- DE-A- 3 914 622
- GB-A- 2 305 604
- US-A- 4 374 082

## Description

The present invention relates to gastro-resistant formulations, preferably tablets, for alimentary or dietary use, which are obtained by mixing the composition with fat in order to achieve a prolonged release of the active principles contained therein to the organism.

The preparation of the gastro-resistant formulations is usually carried out so as to allow the active principle to be released and absorbed in a more or less retarded manner at the intestine level; alternatively, the active principle may be released and absorbed only in part at the stomach level, thus allowing a second fraction of the active principle to be released and absorbed at the intestine level.

The known technique for preparing gastro-resistant formulations with retarded release is as follows:
A) Gastro-resistant formulations: these are tablets lined with gastro-resistant films, such as, for example, ethylcellulose, cellulose acetophthalate, polyacrylates, gum lac, keratine; the lined tablets are then coated with sugar.
B) Layered formulations: they are prepared in the same manner as the gastro-resistant sugar-coated pills, with regard to coating of the tablets; a sprinkling powder such as starch or talcum, in which an active principle is dispersed using a water-soluble product such as gum arabic, agar-agar etc. as the adhesive, is attached layerwise to the coated core, in such a manner that the outermost layer and not the inner tablet is dissolved in the stomach.
C) Capsules containing retarding agents; they are sugar cores in which the active principle is dispersed, followed by application of a protective coating as in para. A);
D) Tablets in which retarding agents are dispersed in such a way that part of the active principle is present in the gastro-resistant retarding agents and part is present in the water-dispersible tablet;
E) Multi-layered tablets in which one or more layers contain dissolution-retarding powders, such as cellulose-derived gum lacs so that the layers have different solubilities.

In general, they are formulations whose retarding effect is based on the use of excipients and/or adjuvants foreign to the mammalian organism, in particular of humans, which formulations are intended to maximize the absorption of the active principle without taking into consideration the normal physiological digestive processes.

However, the use of such substances is usually not very desirable, in particular in the case of dietary formulations and/or in the case of food additives which are intended to achieve instead an absorption of the active principle according to a kinetic profile which is as close as possible to the normal human digestive processes.

The recourse to "natural" absorption profiles is anyway desirable, even in the case of therapeutic formulations, for example in all those classes of patients who would be harmed by administering them non-"physiological" excipients and/or adjuvants; obvious examples are pregnant women, very young children, allergic subjects, etc.

Now, according to the subject-matter of the present invention, a novel fomulation with retarded release has been found, said formulation allowing the active principles to be absorbed utilizing the physiological digestive activity, i.e. imitating what happens with food ingested in the usual manner.

The present invention relates to a formulation in tablet form for oral use, containing at least one active principle with a pharmaceutical, dietary or alimentary action in combination with at least one hydrogenated fatty acid, as the vehicle, in an amount of between 5 und 30%, relative to the weight of the formulation; preferably, such hydrogenated fatty acid is present in an amount of between 20 and 30%, relative to the weight of the formulation.

The hydrogenated fatty acids which can be used for the purposes of the present invention are normally selected from those either of synthetic or natural origin, having a chain comprising between 3 and 20 carbon atoms, preferably between 14 and 18 carbon atoms, and mixtures thereof, hydrogenated palm oil being the preferred one.

As indicated above, the active principles which can be used for the purposes of the present invention may have both a therapeutic and a dietary or alimentary action.

The active principles with a therapeutic action may be selected from among non-steroid anti-inflammatory drugs (NSAID) and steroid anti-inflammatory drugs, tranquilizers, sleeping pills, anti-hypertensive, anti-histaminic and anti-asthmatic drugs; non-steroid anti-inflammatory drugs in turn may be selected from among ibuprofen, naproxen, ketoprofen, indomethacin, acetylsalicylic acid, mefenamic acid, flufenamic acid, etc.; the active principles with a dietary action may be selected from the group consisting of lactic acid microorganisms, beer yeasts, either as such or containing living cells, vitamins, minerals, amino acids, vegetable extracts, and derivatives thereof.

In the formulation according to the present invention, the active principle or principles, which may be used as such or in the form of esters or physiologically acceptable salts, can be mixed directly with said hydrogenated fatty acid without the addition of any excipients and/or adjuvants; in this case, the active principle or principles make up 70-95% by weight, preferably 75-90% by weight, of the formulation.

Alternatively, the abovementioned active principles may be used in combination with customary excipients and/or adjuvants known in the art; in this case, they are normally present in amounts of between 1 and 50%, preferably between 10 and 40%, relative to the total weight of the formulation.

The excipients used for the tablet according to the present invention may be selected from the group consisting of starches, maltodextrin, microcrystalline cellulose, talcum-modified cellulose, calcium carbonate, milk proteins, calcium stearate, magnesium stearate, sodium stearate, soya proteins or suitable inert powders, PVP, precipitated silica and are present in an amount of 10-30% by weight, preferably 20-30% by weight, relative to the total weight of the formulation.

In order to determine the release activity, over time, of an active principle contained in a formulation according to the present invention (the qualitative and quantitative composition of which is given in Example 1), the dissolution test described in Farmacopea Ufficiale Italiana (Official Italian Pharmacopeia) was carried out. The results of said test are shown in the table below.

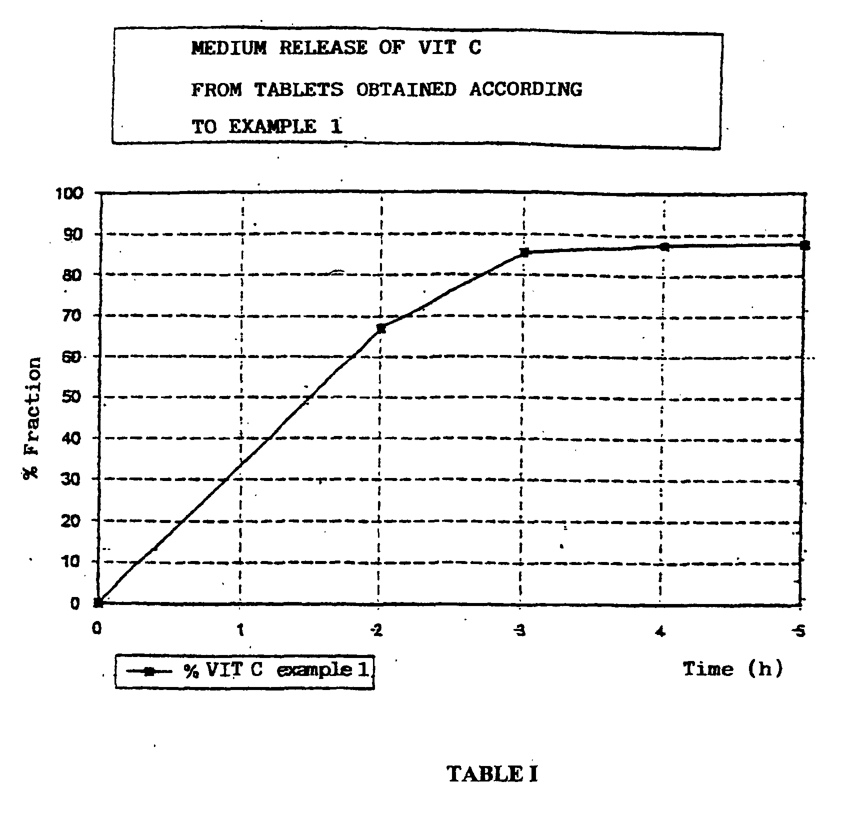

This dissolution test demonstrates the slow release, over time, of an active principle under physiological conditions which simulate the digestive processes which normally take place in the stomach.

The present invention is particularly suitable for the production of BIO-certified gastro-resistant tablets, provided that fats derived from biological cultivations and farms in accordance with current regulations are used.

The present invention furthermore relates to the process for the preparation of the formulations according to the present invention.

Said process comprises premixing an active principle as defined above in an amount of 1-50% by weight, relative to the total weight of the formulation, with the excipients as defined above, which in turn are present in an amount of 10-30%, relative to the total weight of the formulation. The mixture thus obtained by simple mixing at ambient temperature or by dry or wet granulation in accordance with the known technique is kneaded in a suitable kneader, usually a Z-type kneader or plunging-arm kneader, together with at least one hydrogenated fatty acid in the melted state in an amount of between 5 and 30%, relative to the weight of the formulation.

The blend thus obtained is cooled to 5-20° C, preferably to 10°C-12° C, and then granulated, for example using an oscillating granulator of the Manesty type equipped with a perforated stainless steel plate having holes with a diameter of 1-4 mm, preferably 1-2 mm.

The granules thus obtained are compressed with a rotary tablet-compressing machine equipped with suitable punches. It is thus possible to obtain tablets of suitable weight.

In the case of tablets not containing added excipients and/or adjuvants, the active principle is mixed directly with the fat and/or phospholipid in the melted state; the mixture is then processed as described above.

In particular, the present invention is highly suitable for the preparation of layered tablets obtained with a suitable tablet-compressing machine such as, for example, a Manesty BB3B.

The process consists in compressing a layer obtained according to the prior art using one or more active principles mixed with known water-soluble or water-dispersible desired, it is also possible to use more than two layers with different degrees of solubility.

The examples which follow are given in order to describe better the present invention without, however, limiting its scope.

### Example 1

1000 tablets are prepared, being formed by a fast-dissolving layer (Layer A) obtained by kneading, in a Z-type kneader, the following components together with 10% strength Klucel/water:
proline (100 g),
lysine (100 g),
cystine (100 g),
sodium carboxymethylcellulose (20 g)

The blend thus obtained is dried for 12 hours at 40°C in a drying cabinet, the resulting mixture is granulated in a Manesty granulator equipped with a perforated stainless steel plate having holes with a 2-mm diameter, giving a yield of 321.8 g.

The granules thus obtained are mixed in a rotating-screw mixer (SAGA) with :
red lake N° 40 all lake (0.25 g),
vitamin A 5,000,000 IU/g (800 µg/cpr+30%) ( 2.31 g),
vitamin E 50% SD (16 mg/cpr +20%) (12.8 g),
vitamin C granules (49.5 g),
magnesium stearate (5 g),
copper gluconate Cu 14% (1.2 mg/cpr + 5%) (6 g),
zinc gluconate Zn 13.4% (10 mg/cpr + 5%) (52.2 g)
selenium-containing yeast 2,000 µg/g (0.055 µg/cpr + 5%) (19 g)
glutathione on yeast (25 mg/cpr + 20%) (15 g),
rapidly disintegrating PVP (20 g),
potato starch (10 g),
silica gel (3 g),
maltodextrin (5 g),
microcrystalline cellulose (2 g),
water (0.5 g),
giving a total yield of 524.36 g.

A second mixture is prepared and used to form the slow-dissolving layer (LAYER B) thus obtained:
lyophilized blueberry (15 g),
microcrystalline cellulose (50 g),
titanium dioxide (10 g),
nucleic acids (50 g),
blueberry extract 25% (50 g),
copper gluconate (1.5 g),
zinc gluconate (12.3 g),
copper gluconate (1.5 g),
zinc gluconate (13.8 g),
selenium-containing yeast (9.5 g),
glutathione on yeast (15 g),
vitamin A 500,000 IU/g (4.63 g),
vitamin E 50% SD (25.6 g),
vitamin C EC 97% (99 g),

All these components are mixed and kneaded in a Z-type kneader together with melted hydrogenated palm oil (50 g).

The blend obtained is cooled to 12°C and granulated in an oscillating granulator equipped with a stainless steel plate having holes with a 2 mm diameter, giving a total yield of 408 g.

The two mixtures thus obtained can be compressed with an oval punch using a double-layered tablet-compressing machine (MANESTY BB3B) producing oval tablets with a weight of 0.932 g, in which the first layer weighing 0.524 g is fast-dissolving and the second layer weighing 0.408 g is gastro-resistant and slow-dissolving.

### Example 2

Example 1 is repeated, except that the following components are used:
Layer A (FAST-DISSOLVING)
folic acid 98% (0.3 mg/cpr + 20%) (0.12 g)
vitamin B6 33.1/3 (1.5 mg + 20%) (1.8 g)
beta carotene 20% (4mg/cpr + 10%) (7.4 g)
vitamin E 50% SD (116 mg/cpr) (12.8 g)
vitamin C EC 97 (120 mg/cpr+20%) (49.5 g)
copper gluconate Cu 14% (1.2 mg/cpr) (6 g)
zinc gluconate Zn 13.4% (10 mg/cpr) (52.3 g)
selenium-containing yeast 2000 µg/g (55 µg/cpr) (19.3 g)
lactose CD (150 g)
microcrystalline cellulose (30 g)
water (4 g)
potato starch (30 g)
rapidly disintegrating PVP (Kollidon CL) (10 g)
silicagel (10 g)
maltodextrin (8g)giving a total of 391.22 g:
Layer B (SLOW-DISSOLVING)
sulfomucopolysaccharides (25 g)
Gingko biloba (30 g)
copper gluconate Cu 14% (3 g)
zinc gluconate Zn 13.4% (26.2 g)
selenium-containing yeast 2,000 µg/g (9.7 g)
microcrystalline cellulose (50 g)
red iron oxide (5 g)
folic acid (0.24 g)
vitamin B6 33.1/3% (3.6 g)
vitamin E 50% (25.6 g)
vitamin C EC 97% (99 g)
beta carotene 20% (14.8 g)
melted hydrogenated palm oil (72 g)
silica gel (0.5%),
giving a total of 0.358 g.

Double-layered tablets weighing 0.749 g are prepared, the first layer of which weighing 0.391 g is fast-dissolving and the second one weighing 0.358 g is slow-dissolving.

The tablets can then be coated with a solution of
10 % strength Klucel/water.

### Example 3

Example 1 is repeated, except that the following components are used:

| Layer A (FAST-DISSOLVING) | |
|---|---|
| acetylsalicylic acid | 0.3 g |
| hydrogenated palm oil | 0.1 g |
| lactose | 0.2 g |

| Layer B (SLOW-DISSOLVING) | |
|---|---|
| acetylsalicylic acid | 0.2 g |
| lactose | 0.1 g |
| magnesium stearate | 0.01 g |
| pre-dried corn starch | 0.1 g |

## Claims

1. Process for the preparation of a retarded release formulation for oral use in tablet form, containing at least one active principle with a pharmaceutical, dietary or alimentary action and at least one hydrogenated fatty acid, as the vehicle, in amounts of between 5 and 30%, relative to the weight of the formulation, wherein:
(a) said at least one active principle is mixed with said at least one hydrogenated fatty acid in the melted state in the weight proportions defined above;
(b) the blend thus obtained is cooled to 5-20°C and then granulated using a granulator having holes with a diameter of between 1 and 4 mm;
(c) the granules thus obtained are then compressed.

2. The process according to claim 1, wherein said hydrogenated fatty acid is present in amounts between 10 and 20%, relative to the weight of the formulation.

3. The process according to claims 1-2, wherein the blend in point (b) is cooled to 10°C-12°C.

4. The process according to claims 1-3, wherein the blend in point (b) is granulated using a granulator having holes with a diameter of between 1 and 2 mm.

5. A formulation obtainable by the process according to claims 1-4.

6. A formulation according to claim 5, **characterized in that** said at least one hydrogenated fatty acid has a chain comprising between 3 and 20 carbon atoms, preferably between 14 and 18 carbon atoms, or mixtures thereof.

7. A formulation according to claims 5-6, **characterized in that** said at least one hydrogenated fatty acid is hydrogenated palm oil.

8. A formulation according to claims 5-7, **characterized in that** said at least one active principle is present in an amount of 70-95%, preferably 75-90%, relative to the weight of the formulation, and **in that** said at least one active principle and said at least one hydrogenated fatty acid make up 100% by weight of the formulation.

9. A formulation according to claims 5-8, **characterized by** containing: (a) from 10 to 50% by weight, of at least one active principle with a pharmaceutical, dietary or alimentary action; (b) from 5 to 30% by weight of at least one hydrogenated fatty acid and (c) exicipients and/or adjuvants, the sum of the components (a), (b) and (c) making up 100% by weight of the formulation.

10. A formulation according to claim 9, **characterized by** containing from 30 to 50% by weight of component (a).

11. A formulation according to claims 9-10, **characterized by** containing from 20 to 30% by weight of component (b).

12. A formulation according to claims 9-11, **characterized in that** said at least one active principle with a therapeutic action is selected from non-steroid and steroid anti-inflammatory drugs, tranquilizers, sleeping pills, anti-hypertensive, anti-histaminic and anti-asthmatic drugs and **in that** said at least one active principle with a dietary or alimentary action is selected from the group consisting of lactic acid microorganisms, beer yeasts, either as such or containing living cells, vitamins, minerals, amino acids, vegetable extracts, and derivatives thereof.

13. A formulation according to anyone of the preceeding claims **characterized by** being a layered tablet.

## Patentansprüche

1. Verfahren zur Herstellung einer Formulierung mit verzögerter Freisetzung zur oralen Verwendung in Tablettenform, die wenigstens einen Wirkstoff mit pharmazeutischer, diätetischer oder Ernährungswirkung und wenigstens eine hydrierte Fettsäure als Träger in Mengen zwischen 5 und 30 % relativ zum Gewicht der Formulierung enthält, worin:
(a) der wenigstens eine Wirkstoff mit der wenigstens einen hydrierten Fettsäure im geschmolzenen Zustand in den oben definierten Gewichtsanteilen vermischt wird;
(b) die so erhaltene Mischung auf 5 bis 20°C abgekühlt und dann unter Verwendung eines Granulators mit Löchern mit einem Durchmesser zwischen 1 und 4 mm granuliert wird;
(c) die so erhaltenen Granalien dann verpreßt werden.

2. Verfahren gemäß Anspruch 1, worin die hydrierte Fettsäure in Mengen zwischen 10 und 20 % relativ zum Gewicht der Formulierung vorliegt.

3. Verfahren gemäß den Ansprüchen 1 bis 2, worin die Mischung in Ziffer (b) auf 10 bis 12°C abgekühlt wird.

4. Verfahren gemäß den Ansprüchen 1 bis 3, worin die Mischung in Ziffer (b) unter Verwendung eines Granulators mit Löchern mit einem Durchmesser zwischen 1 und 2 mm granuliert wird.

5. Formulierung, die durch das Verfahren gemäß den Ansprüchen 1 bis 4 erhältlich ist.

6. Formulierung gemäß Anspruch 5, **dadurch gekennzeichnet, daß** die wenigstens eine hydrierte Fettsäure eine Kette, die zwischen 3 und 20 Kohlenstoffatomen umfaßt, bevorzugt zwischen 14 und 18 Kohlenstoffatomen, oder Mischungen daraus umfaßt.

7. Formulierung gemäß den Ansprüchen 5 bis 6, **dadurch gekennzeichnet, daß** die wenigstens eine hydrierte Fettsäure hydriertes Palmöl ist.

8. Formulierung gemäß den Ansprüchen 5 bis 7, **dadurch gekennzeichnet, daß** der wenigstens eine Wirkstoff in einer Menge von 70 bis 95 %, bevorzugt 75 bis 90 % relativ zum Gewicht der Formulierung vorliegt, und daß der wenigstens eine Wirkstoff und die wenigstens eine hydrierte Fettsäure 100 Gew.-% der Formulierung darstellen.

9. Formulierung gemäß den Ansprüchen 5 bis 8, **dadurch gekennzeichnet, daß** sie (a) 10 bis 50 Gew.-% wenigstens eines Wirkstoffs mit einer pharmazeutischen, diätetischen oder Ernährungswirkung; (b) 5 bis 30 Gew.-% wenigstens einer hydrierten Fettsäure und (c) Exzipienten und/oder Hilfsstoffe enthält, wobei die Summe der Komponenten (a), (b) und (c) 100 Gew.-% der Formulierung darstellt.

10. Formulierung gemäß Anspruch 9, **dadurch gekennzeichnet, daß** sie 30 bis 50 Gew.-% von Komponente (a) enthält.

11. Formulierung gemäß den Ansprüchen 9 bis 10, **dadurch gekennzeichnet, daß** sie 20 bis 30 Gew.-% von Komponente (b) enthält.

12. Formulierung gemäß den Ansprüchen 9 bis 11, **dadurch gekennzeichnet, daß** der wenigstens eine Wirkstoff mit einer therapeutischen Wirkung aus nicht-steroidalen und steroidalen entzündungshemmenden Wirkstoffen, Beruhigungsmitteln, Schlaftabletten, Antihypertonika, Antihistaminika und Antiasthmatika ausgewählt ist, und daß der wenigstens eine Wirkstoff mit einer diätetischen oder Ernährungswirkung aus der Gruppe ausgewählt ist, die aus Milchsäure-Mikroorganismen, Bierhefen, entweder als solche oder lebende Zellen enthaltend, Vitaminen, Mineralien, Aminosäuren, Pflanzenextrakten und Derivaten davon besteht.

13. Formulierung gemäß einem der vorhergehenden Ansprüche, **dadurch gekennzeichnet, daß** sie eine Schichttablette ist.

## Revendications

1. Procédé de préparation d'une formulation à déblocage libération retardée pour une utilisation orale sous forme de comprimés, contenant au moins un principe actif ayant une action alimentaire, diététique ou thérapeutique et au moins un acide gras hydrogéné, pour le véhicule, en quantités comprises entre 5 et 30 % par rapport au poids de la formulation, dans lequel :
(a) ledit, au moins un, principe actif est mélangé avec ledit, au moins un, acide gras hydrogéné à l'état fondu dans les proportions de poids définies ci-dessus ;
(b) le mélange ainsi obtenu est refroidi à 5-20°C et ensuite transformé en granulés au moyen d'un granulateur ayant des orifices avec un diamètre compris entre 1 et 4 mm ;
(c) les granulés ainsi obtenus sont alors comprimés.

2. Procédé selon la revendication 1, dans lequel ledit acide gras hydrogéné est présent en quantités comprises entre 10 et 20 % par rapport au poids de la formulation.

3. Procédé selon les revendications 1 et 2, dans lequel le mélange au point (b) est refroidi à 10°C-12°C.

4. Procédé selon les revendications 1 à 3, dans lequel le mélange au point (b) est transformé en granulés au moyen d'un granulateur ayant des orifices avec un diamètre compris entre 1 et 2 mm.

5. Formulation pouvant être obtenue à partir du procédé selon les revendications 1 à 4.

6. Formulation selon la revendication 5, **caractérisée en ce que** ledit, au moins un, acide gras hydrogéné a une chaîne comprenant entre 3 et 20 atomes de carbone, de préférence entre 14 et 18 atomes de carbone ou des mélanges de ceux-ci.

7. Formulation selon les revendications 5 et 6, **caractérisée en ce que** ledit, au moins un, acide gras hydrogéné est une huile de palme hydrogénée.

8. Formulation selon les revendications 5 à 7, **caractérisée en ce que** ledit, au moins un, principe actif est présent en quantité de 70-95 %, de préférence 75-90 %, par rapport au poids de la formulation et **en ce que** ledit, au moins un, principe actif et ledit au moins un acide gras hydrogéné réalisent 100 % en poids de la formulation.

9. Formulation selon les revendications 5 à 8, **caractérisée en ce qu'**elle contient : (a) entre 10 et 50 % en poids d'au moins un principe actif ayant une action alimentaire, diététique ou thérapeutique ; (b) entre 5 et 30 % en poids d'au moins un acide gras hydrogéné et (c) des excipients et/ou des adjuvants, la somme des composants (a), (b) et (c) réalisant 100 % en poids de la formulation.

10. Formulation selon la revendication 9, **caractérisée en ce qu'**elle contient entre 30 et 50 % en poids du composant (a).

11. Formulation selon les revendications 9 et 10, **caractérisée en ce qu'**elle contient entre 20 et 30 % en poids du composant (b).

12. Formulation selon les revendications 9 à 11, **caractérisée en ce que** ledit, au moins un, principe actif ayant une action thérapeutique est choisi parmi les médicaments anti-inflammatoires stéroïdiens et non-stéroïdiens, les tranquillisants, les hypnotiques, les médicaments anti-hypertenseurs, anti-histaminiques et anti-asthmatiques et **en ce que** ledit, au moins un, principe actif ayant une action alimentaire ou diététique est choisi parmi le groupe composé de micro-organismes d'acide lactique, de levures de bière, soit tels quels soit contenant des cellules vivantes, des vitamines, des minéraux, des acides aminés, des extraits végétaux, et des dérivés de ceux-ci.

13. Formulation selon l'une quelconque des revendications précédentes, **caractérisée en ce qu'**il s'agit d'un comprimé multicouches.
